# EUROPEAN PATENT APPLICATION

(11) **EP 4 119 936 A1**
(43) Date of publication of application: **18.01.2023**
(21) Application number: 21767269.0
(22) Date of filing: 27.01.2021
(51) Int. Cl.: G01N 27/26, G01N 27/416

(54) **CALIBRATION LIQUID FOR WATER QUALITY MEASUREMENT DEVICE, CALIBRATION-LIQUID-FILLED CONTAINER FOR WATER QUALITY MEASUREMENT DEVICE, AND CALIBRATION METHOD USING CALIBRATION LIQUID FOR WATER QUALITY MEASUREMENT DEVICE**

(30) Priority: 09.03.2020 JP 2020039707
(71) Applicant: NGK SPARK PLUG CO., LTD., Mizuho-ku Nagoya-shi Aichi 467-8525 (JP)
(72) Inventor: KAMEI, Shunsuke, Nagoya-shi, Aichi 467-8525 (JP); TASHIMA, Keisuke, Nagoya-shi, Aichi 467-8525 (JP); OSAWA, Norimasa, Nagoya-shi, Aichi 467-8525 (JP); TAMAI, Kazusei, Nagoya-shi, Aichi 467-8525 (JP); KOJIMA, Junji, Kizugawa-shi, Kyoto 619-0223 (JP); IWAMOTO, Yasukazu, Kizugawa-shi, Kyoto 619-0223 (JP); NAGAI, Hiroshi, Kizugawa-shi, Kyoto 619-0223 (JP)
(74) Representative: Grünecker Patent- und Rechtsanwälte PartG mbB
(86) International application number: PCT/JP2021/002772
(87) International publication number: WO 2021/181922

(57) **Abstract**

A calibration liquid (1) is used for calibrating a water quality measurement device (100). The water quality measurement device (100) includes a plurality of sensors to be brought into contact with a measurement target liquid so as to measure concentrations of a plurality of types of target components contained in the measurement target liquid, and measures the concentrations of the plurality of types of target components. The calibration liquid (1) includes a solution which contains the plurality of types of target components at respective concentrations determined for the target components, and the solution has a pH within a predetermined pH range.

## Description

### TECHNICAL FIELD

The present invention relates to a calibration liquid for water quality measurement device, a calibration-liquid-filled container for water quality measurement device, and a calibration method using a calibration liquid for water quality measurement device.

### BACKGROUND ART

A land-based aquaculture apparatus disclosed in Patent Literature 1 includes an aquaculture tank filled with aquaculture water (seawater) for culturing fishery products, an ammonia sensor for detecting the ammonia concentration in the water, and an ion sensor for detecting the concentration of a specific ion in the water. The ion sensor is, for example, a nitrate ion sensor. An ion electrode for detecting nitrate ion and a reference electrode are connected so as to measure an electromotive force between the two electrodes in a test solution, thereby calculating the concentration of nitrate ion.

### CITATION LIST

### PATENT LITERATURE

Patent Literature 1: JP2003-052275A

### SUMMARY OF INVENTION

### TECHNICAL PROBLEM

Incidentally, a device for electrically measuring the concentration of a certain ion to be measured (hereinafter referred to the target ion) must be calibrated on the basis of calibration liquid so as to accurately measure the concentration of the target ion. In such a case, it is necessary to use a dedicated calibration liquid which contains the target ion and whose target ion concentration has been specified accurately.

Some water quality measurement devices can detect the concentrations of a plurality of types of ions contained in a measurement target liquid. Such devices must be calibrated for each ion type so as to accurately measure the concentration of each target ion. A conceivable method for such calibration is to use a dedicated calibration liquid for each target ion. However, when this calibration method is employed, the greater the number of the types of target ions, the greater the number of the types of dedicated calibration liquids needed for calibration, which results in an increase in management burden. In addition, in this calibration method, the respective dedicated calibration liquids must be fed sequentially so as to sequentially perform calibration operations corresponding to the respective target ions. Therefore, the greater the number of the types of target ions, the greater the number of times the calibration liquids are fed, which results in an increase in time necessary for the calibration.

The present invention has been accomplished so as to at least partially solve the above-described problem and its object is to reduce burden associated with management of calibration liquid and shorten calibration time.

### SOLUTION TO PROBLEM

A calibration liquid for water quality measurement device, which is one solution of the present invention, is used for calibration of a water quality measurement device which includes a plurality of sensors to be brought into contact with a measurement target liquid so as to measure concentrations of a plurality of types of target components contained in the above-described measurement target liquid and which measures the concentrations of the above-described plurality of types of target components. The calibration liquid is used for calibrating characteristics of the water quality measurement device for the concentrations of the above-described plurality of types of target components. The calibration liquid includes a solution which contains the above-described plurality of types of target components at respective concentrations determined for the above-described target components, and the above-described solution has a pH within a predetermined pH range.

The above-described calibration liquid enables performance of calibration for the plurality of types of target components by using a common solution. Therefore, when the water quality measurement device is calibrated, the above-described calibration liquid can reduce management burden resulting from preparation of a dedicated calibration liquid for each target component. In addition, use of the above-described calibration liquid can easily shorten calibration time, as compared with a method in which dedicated calibration liquids for the respective target components are sequentially fed to the water quality measurement device so as to sequentially perform calibration operations corresponding to the respective target components.

In the calibration liquid for water quality measurement device, which is a first aspect of the present invention, one or more types of target components among the plurality of types of target components may be gas formation ions for which pH stable ranges are determined such that, when the pH of liquid containing each gas formation ion falls outside the corresponding pH stable range, transformation of the gas formation ion to a gas is promoted. The pH of the solution may be set to fall within the pH stable ranges of all types of the gas formation ions contained in the solution.

The above-described calibration liquid enables performance of calibration for the plurality of types of target components, and the above-described gas formation ions can be included therein. In addition, in the above-described calibration liquid, the pH of the solution is set to fall within the pH stable ranges of all types of the gas formation ions contained in the solution. Therefore, the above-described calibration liquid is stably held in a state in which transformation of the gas formation ions to gases is suppressed.

In the calibration liquid for water quality measurement device, which is the first aspect of the present invention, one or more types of target components among the plurality of types of target components may be compound formation ions for which pH stable ranges are determined such that, when the pH of liquid containing each compound formation ion falls outside the corresponding pH stable range, transformation of the compound formation ion to a compound is promoted. The pH of the solution may be set to fall within the pH stable ranges of all types of the compound formation ions contained in the solution.

The above-described calibration liquid enables performance of calibration for the plurality of types of target components, and the above-described compound formation ions can be included therein. In addition, in the above-described calibration liquid, the pH of the solution is set to fall within the pH stable ranges of all types of compound formation ions contained in the solution. Therefore, the above-described calibration liquid is stably held in a state in which transformation of the compound formation ions to compounds is suppressed.

Furthermore, in the case where the above-described plurality of types of target components include not only the above-described compound formation ions but also the above-described gas formation ions, desirably, the pH of the solution is set to fall within the pH stable ranges of all types of compound formation ions and fall within the pH stable ranges of all types of gas formation ions. This calibration liquid prepared in this manner is stably held in a state in which transformation of the target components to gases and transformation of the target components to compounds are suppressed in an environment in which the gas formation ions and the compound formation ions coexist in the solution.

In the calibration liquid for water quality measurement device, which is the first aspect of the present invention, the solution may include at least a first solution and a second solution which are different solutions. The first solution and the second solution may contain the plurality of types of target components as common target components. The concentrations of the common target components contained in the first solution may differ from the concentrations of the common target components contained in the second solution. The pH of the first solution and the pH of the second solution may differ from each other.

In the case where the water quality measurement device performs measurement for the first solution of the above-described calibration liquid, the water quality measurement device can accurately specify the relation between the concentration of each of the target components contained in the first solution and an electrical signal (for example, a voltage signal) output from the corresponding sensor. Furthermore, in the case where the water quality measurement device performs measurement for the second solution, the water quality measurement device can accurately specify the relation between the concentration of each of the target components contained in the second solution and an electrical signal (for example, a voltage signal) output from the corresponding sensor. Therefore, the above-described calibration liquid enables the water quality measurement device to accurately obtain a relational expression representing the relation between the concentration of each target component and the corresponding electrical signal.

In the calibration liquid for water quality measurement device, which is the first aspect of the present invention, the solution may contain at least sodium ion, chloride ion, and potassium ion. The target components may include components other than sodium ion, chloride ion, and potassium ion.

The above-described calibration liquid enables calibration for a plurality of types of target components by using a solution which contains seawater or components of seawater.

A calibration-liquid-filled container for water quality measurement device, which is a second aspect of the present invention, may comprise any of the above-described calibration liquids and a container body for containing the calibration liquid, wherein the calibration liquid is contained in the container body, and the container body is tightly sealed.

The above-described calibration-liquid-filled container can reduce burden associated with management of the calibration liquid and can shorten calibration time. In addition, the above-described calibration-liquid-filled container enables management of the calibration liquid, containing a plurality of types of components in a mixed state, in a container body having a high sealing performance.

A calibration method, which is a third aspect of the present invention, is a calibration method for a water quality measurement device which measures concentrations of a plurality of types of target components contained in a measurement target liquid, the calibration method calibrating characteristics of the water quality measurement device for the concentrations of the plurality of types of target components, wherein the calibration method uses any of the above-described calibration liquids.

The above-described calibration method enables performance of calibration for the plurality of types of target components by using a common solution. Therefore, when the water quality measurement device is calibrated, the above-described calibration method can reduce management burden resulting from preparation of dedicated calibration liquid for each target component. In addition, the above-described calibration method can easily shorten calibration time, as compared with a method in which dedicated calibration liquids for the respective target components are sequentially fed to the water quality measurement device so as to sequentially perform calibration operations corresponding to the respective target components.

### ADVANTAGEOUS EFFECTS OF INVENTION

The present invention can reduce burden associated with management of calibration liquid and shorten calibration time.

### BRIEF DESCRIPTION OF DRAWINGS

[FIG. 1] FIG. 1 is a block diagram which schematically illustrates a water quality measurement device in which a calibration liquid of a first embodiment is used.
[FIG. 2] FIG. 2 is an explanatory view used for describing a first sensor section of the water quality measurement device of FIG. 1.
[FIG. 3] FIG. 3 is an explanatory view used for describing a second sensor section of the water quality measurement device of FIG. 1.
[FIG. 4] FIG. 4 is a schematic view schematically showing a calibration-liquid-filled container which contains the calibration liquid of the first embodiment.
[FIG. 5] FIG. 5 is an explanatory view used for describing a method for performing calibration for target components.
[FIG. 6] FIG. 6 is an explanatory view used for describing a method for performing calibration for target components.

### DESCRIPTION OF EMBODIMENTS

### <First embodiment>

### 1. Regarding water quality measurement device

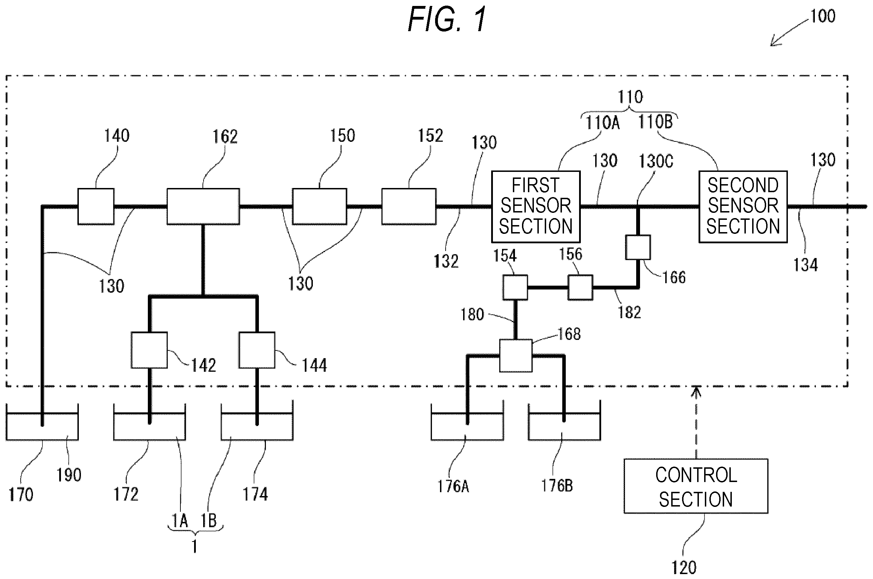

A water quality measurement device 100 in which a calibration liquid 1 of a first embodiment is used is illustrated in FIG. 1.

The water quality measurement device 100 of FIG. 1 may be a device for measuring the quality of breeding water for breeding a creature (for example, breeding water used in the field of aquaculture or the like) as a liquid whose quality is to be measured (hereinafter referred to as "target liquid") or a device for measuring the quality of a target liquid of a type different from breeding water. The water quality measurement device 100 may be one used for water quality management in an aquaculture facility, an aquarium, a hydroponic culture facility, or the like, one used for water quality management in other facilities, or one used for water quality management outside the facilities. The measurement target liquid 190 shown in FIG. 1 may be, for example, water containing salt content (for example, seawater, brackish water, artificial seawater, or the like), water containing no salt content, or water which is low in salinity.

The water quality measurement device 100 is a device for measuring the concentrations of target contents contained in the measurement target liquid. Methods by which the water quality measurement device 100 measures the concentrations of the target components include a method which directly measures the concentrations of target components (for example, target ions such as calcium ion, magnesium ion, and the like) contained in the measurement target liquid and a method which measures the concentrations of gas components (for example, nitrous acid gas and ammonia gas) formed from target components (for example, target ions such as nitrite ion, ammonium ion, and the like) contained in the measurement target liquid. In the example of FIG. 1, the water quality measurement device 100 is configured such that the measurement target liquid is contained in a containing section 170 automatically or manually, and the water quality measurement device 100 supplies the measurement target liquid 190 contained in the containing section 170 to a sensor section 110 for measurement of ion concentrations.

The water quality measurement device 100 includes containing sections 170, 172, and 174, a supply passage 130, electromagnetic valves 140, 142, 144, and 166, three-way valves 162 and 168, pumps 150 and 154, flow rate sensors 152 and 156, a control section 120, etc.

The containing section 170 is configured to serve as a sampling container for containing the measurement target liquid. The containing section 172 is configured to serve as a container for containing a first solution 1A of a calibration liquid 1. The containing section 174 is configured to serve as a container for containing a second solution 1B of the calibration liquid 1. The containing section 176A is a container for containing an acid (for example, sulfuric acid). The containing section 176B is a container for containing a base (for example, aqueous sodium hydroxide).

The supply passage 130 is a flow passage configured to feed the measurement target liquid 190 from the containing section 170 to the sensor section 110 and drain the measurement target liquid 190 from the sensor section 110. A flow passage portion of the supply passage 130, which portion is disposed on the upstream side of the first sensor section 110A to be the closest thereto and is configured to introduce liquid to the first sensor section 110A, is an introduction passage 132. A flow passage portion of the supply passage 130, which portion is disposed between the first sensor section 110A and the second sensor section 110B, is an intermediate passage 133. A flow passage portion of the supply passage 130, which portion is disposed on the downstream side of the second sensor section 110B to be the closest thereto and is configured to drain liquid from the second sensor section 110B, is a drain passage 134.

The control section 120 is configured in the form of an information processing device and has a function for performing various types of controls. The control section 120 also has a function for obtaining various signals and a function for performing various types of information processing.

The electromagnetic valve 140 is an electromagnetic valve which opens and closes in accordance with the control performed by the control section 120. The electromagnetic valve 140 is switched between a state in which the electromagnetic valve 140 permits flow at a predetermined position of the supply passage 130 (specifically, a position on the upstream side of the first sensor section 110A) and a state in which the electromagnetic valve 140 interrupts the flow. Each of the electromagnetic valves 142 and 144 is a valve which is switched between a state in which the valve permits flow between a corresponding one of the containing sections 172 and 174 and the three-way valve 162 and a state in which the valve interrupts the flow.

The three-way valve 162 is controlled by the control section 120. The three-way valve 162 is a valve which can switch a source for feeding liquid to a portion of the supply passage 130 located on the downstream side of the three-way valve 162; i.e., can switch the source between the containing section 170 side and the side where the containing sections 172 and 174 are provided. When the three-way valve 162 is in a first changeover state, supply of liquid from the containing section 170 side to the sensor section 110 side is permitted, and supply of liquid from the side where the containing sections 172 and 174 are provided to the sensor section 110 side is interrupted. When the three-way valve 162 is in a second changeover state, supply of liquid from the side where the containing sections 172 and 174 are provided to the sensor section 110 side is permitted, and supply of liquid from the containing section 170 side to the sensor section 110 side is interrupted.

The electromagnetic valve 166 is controlled by the control section 120. The electromagnetic valve 166 is switched between an open state in which the electromagnetic valve 166 permits flow of liquid between the supply passage 182 and the supply passage 130 and a closed state in which the electromagnetic valve 166 does not permit the flow. When the electromagnetic valve 166 is in the closed state, flow of liquid between the supply passage 182 and the supply passage 130 is interrupted, whereby supply of an acid or a base from the supply passage 182 side to the supply passage 130 is interrupted. When the electromagnetic valve 166 is in the open state, the supply of liquid from the supply passage 182 side to the supply passage 130 side is permitted. A merging portion 130C is a portion where a supply passage extending from the electromagnetic valve 166 side is connected to the supply passage 130 and where the liquid flowing from the electromagnetic valve 166 side can merge into the liquid flowing through the supply passage 130.

The three-way valve 168 is controlled by the control section 120. The three-way valve 168 is a valve which can switch a source for feeding liquid to the supply passage 180; i.e., can switch the source between the containing section 176A side and the containing section 176B side. When the three-way valve 168 is in a first changeover state, the three-way valve 168 permits flow of liquid between the containing section 176A and the supply passage 180 and interrupts flow of liquid between the containing section 176B and the supply passage 180, thereby allowing supply of the acid contained in the containing section 176A to the supply passage 180. When the three-way valve 168 is in a second changeover state, the three-way valve 168 permits flow of liquid between the containing section 176B and the supply passage 180 and interrupts flow of liquid between the containing section 176A and the supply passage 180, thereby allowing supply of the base contained in the containing section 176B to the supply passage 180.

The supply passage 180 is a path through which the liquid supplied from the three-way valve 168 flows, and its one end is connected to the three-way valve 168. The other end of the supply passage 180 is connected to the pump 154. The supply passage 182 is a path for supplying to the electromagnetic valve 166 side the liquid supplied to the pump 154 side via the supply passage 180.

The pump 150, which is a well-known pump, is provided in the supply passage 130. The pump 150 has a function of feeding out the liquid in the supply passage 130 to the downstream side when the pump 150 is operating. Specifically, the pump 150 has a function of feeding out the liquid on the three-way valve 162 side to the sensor section 110 side when the pump 150 is operating. The pump 154, which is a well-known pump, is provided between the supply passage 180 and the supply passage 182. The pump 154 has a function of feeding out the liquid in the supply passage 180 to the supply passage 182 side (the electromagnetic valve 166 side) when the pump 154 is operating.

A flow-rate sensor 152 is a sensor for measuring the flow rate of the liquid flowing through the supply passage 130. A flow-rate sensor 156 is a sensor for measuring the flow rate of the liquid flowing through the supply passage 182.

In the case where the control section 120 performs ion concentration measurement for the measurement target liquid 190, the control section 120 brings the three-way valve 162 into the above-described first changeover state, switches the electromagnetic valve 140 to its open state, and activates the pump 150. As a result, the supply of liquid to the sensor section 110 side from the containing section 170 side is permitted, and the supply of liquid to the sensor section 110 from the side where the containing sections 172 and 174 are provided is interrupted, whereby the measurement target liquid 190 contained in the containing section 170 is fed to the sensor section 110 via the supply passage 130. The sensor section 110 performs concentration measurement for the measurement target liquid 190 fed to the sensor section 110 in the above-described manner.

In the case where the control section 120 performs calibration by using the first solution 1A, the control section 120 brings the three-way valve 162 into the above-described second changeover state, the switches the electromagnetic valve 142 to its open state, switches the electromagnetic valves 140 and 144 to their closed states, and activates the pump 150. As a result, in a state in which the supply of liquid from the containing sections 170 and 174 to the sensor section 110 is interrupted, the first solution 1A is supplied from the containing section 172 to the sensor section 110, whereby the first solution 1A contained in the containing section 172 is fed to the sensor section 110. The sensor section 110 performs concentration measurement for the first solution 1A fed to the sensor section 110 in the above-described manner.

In the case where the control section 120 performs calibration by using the second solution 1B, the control section 120 brings the three-way valve 162 into the above-described second changeover state, switches the electromagnetic valve 144 to its open state, switches the electromagnetic valves 140 and 142 to their closed states, and activates the pump 150. As a result, in a state in which the supply of liquid from the containing sections 170 and 172 to the sensor section 110 is interrupted, the second solution 1B is supplied from the containing section 174 to the sensor section 110, whereby the second solution 1B contained in the containing section 174 is fed to the sensor section 110. The sensor section 110 performs concentration measurement for the second solution 1B fed to the sensor section 110 in the above-described manner.

Notably, although not illustrated, a filtration section for performing filtration treatment on the liquid flowing through the supply passage 130 may be provided in the supply passage 130. This filtration section may be configured to remove foreign substances from the liquid flowing through the supply passage 130.

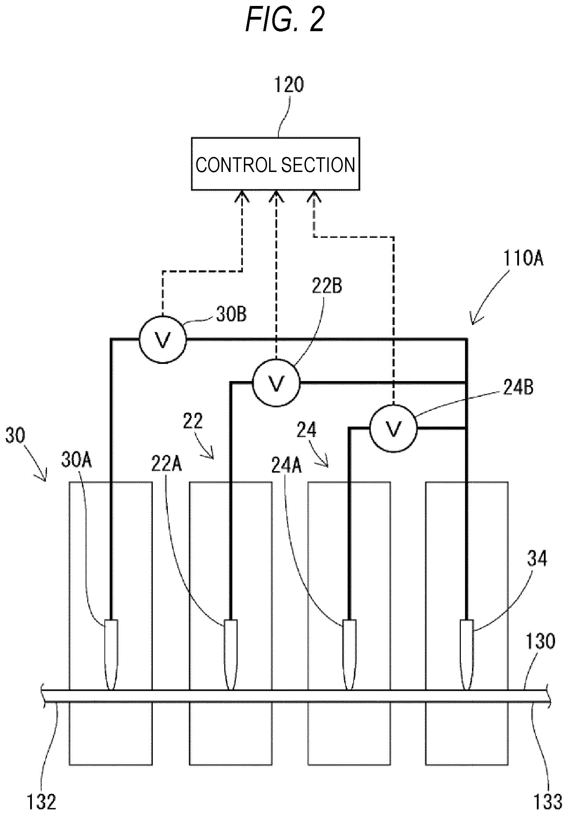

The internal structure of the first sensor section 110A is schematically shown in FIG. 2.

Ions (target ions) whose concentrations are to be measured at the first sensor section 110A are determined in advance. The first sensor section 110A measures the concentrations of a plurality of types of target ions contained in the liquid flowing into the sensor section 110 via the supply passage 130. In the case where the measurement target liquid 190 is supplied from the containing section 170 to the sensor section 110, "the liquid flowing into the sensor section 110" is the measurement target liquid 190. In the case where the first solution 1A is supplied from the containing section 172 to the sensor section 110, "the liquid flowing into the sensor section 110" is the first solution 1A. In the case where the second solution 2A is supplied from the containing section 174 to the sensor section 110, "the liquid flowing into the sensor section 110" is the second solution 2A. Notably, in the following description, the liquid flowing into the sensor section 110 via the supply passage 130 will be also referred to as "supplied liquid." In the example of FIG. 1, the target ions are four types of ions; i.e., calcium ion, magnesium ion, nitrite ion, and ammonium ion.

As shown in FIG. 2, the first sensor section 110A includes a pH sensor 30, a calcium ion sensor 22, a magnesium ion sensor 24, and a reference electrode 34.

The pH sensor 30 shown in FIG. 2 is configured as a pH sensor which operates in accordance with a well-known principle. The pH sensor 30 is provided in the supply passage 130 to be located on the upstream side of the calcium ion sensor 22. More specifically, the pH sensor 30 is provided in the first sensor section 110A to be located on the upstream side of the calcium ion sensor 22. The pH sensor 30 includes an electrode portion 30A and a potentiometer 30B. The electrode portion 30A is a glass electrode. When the supplied liquid (for example, the measurement target liquid 190 (FIG. 1)) fills the supply passage 130, the surface of the electrode portion 30A is immersed in the supplied liquid and comes into contact with the supplied liquid. At that time, the pH sensor 30 generates, at the electrode portion 30A, a potential corresponding to the pH of the supplied liquid in the vicinity of the electrode portion 30A. The potentiometer 30B measures the difference between the potential of the electrode portion 30A and the potential of the reference electrode 34 and outputs to the control section 120 a signal indicating the potential difference (a voltage signal corresponding to the pH of the supplied liquid).

The reference electrode 34 shown in FIG. 2 is a well-known reference electrode (comparative electrode) and generates a reference potential which serves as a reference for the electrode potentials of the pH sensor 30, the calcium ion sensor 22, the magnesium ion sensor 24, and the pH sensor 32. In the example shown in FIG. 2, the reference electrode 34 is provided in the supply passage 130 to be located on the downstream side of the magnesium ion sensor 24 and on the upstream side of the intermediate passage 133.

The calcium ion sensor 22 is provided in the supply passage 130 to be located on the downstream side of the introduction passage 132 (specifically, on the downstream side of the pH sensor 30). The calcium ion sensor 22 includes an ion electrode 22A and a first potentiometer 22B. The ion electrode 22A is, for example, a known liquid-membrane-type ion electrode. The ion electrode 22A includes an electrode film which selectively reacts with calcium ion and is configured to generate an electromotive force corresponding to the calcium ion concentration of the supplied liquid. The ion electrode 22A is immersed into the supplied liquid which flows through the supply passage 130 on the downstream side of the introduction passage 132 (specifically, on the downstream side of the pH sensor 30). The ion electrode 22A generates an electrode potential corresponding to the concentration of calcium ion contained in the supplied liquid in the vicinity of the ion electrode 22A. The first potentiometer 22B is electrically connected to the ion electrode 22A and the reference electrode 34. The first potentiometer 22B measures the potential difference between the electrode potential of the ion electrode 22A and the electrode potential of the reference electrode 34 and outputs to the control section 120 a signal indicating the potential difference (a signal indicating the potential difference corresponding to the concentration of calcium ion contained in the supplied liquid; i.e., the liquid supplied to the sensor section 110).

The magnesium ion sensor 24 is provided in the supply passage 130 to be located on the downstream side of the calcium ion sensor 22. The magnesium ion sensor 24 includes an ion electrode 24A and a second potentiometer 24B. The ion electrode 24A is, for example, a liquid-membrane-type ion electrode. The ion electrode 24A is immersed into the supplied liquid which flows through the supply passage 130 on the downstream side of the ion electrode 22A. The ion electrode 24A generates an electrode potential corresponding to the concentration of magnesium ion contained in the supplied liquid having passed the calcium ion sensor 22. The second potentiometer 24B is electrically connected to the ion electrode 24A and the reference electrode 34. The second potentiometer 24B measures the potential difference between the electrode potential of the ion electrode 24A and the electrode potential of the reference electrode 34 and outputs to the control section 120 a signal indicating the potential difference (a signal indicating the potential difference corresponding to the concentration of magnesium ion contained in the supplied liquid; i.e., the liquid supplied to the sensor section 110).

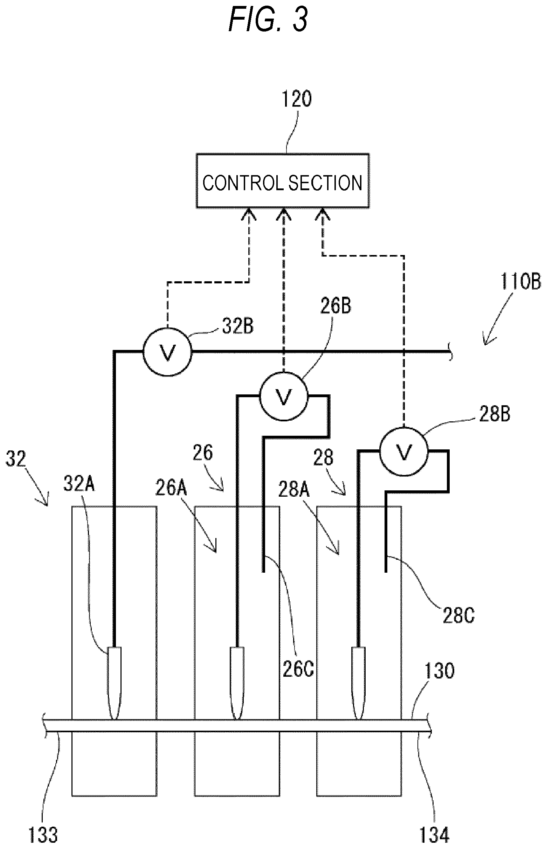

As shown in FIG. 3, the second sensor section 110B includes a pH sensor 32, an ammonium ion sensor (ammonia sensor) 26, and a nitrite ion sensor (nitrous acid sensor) 28.

The pH sensor 32 shown in FIG. 3 has the same configuration as the pH sensor 30 (FIG. 2) and functions in the same manner. When the supplied liquid (for example, the measurement target liquid 190 (FIG. 1)) fills the supply passage 130, the pH sensor 32 generates, at the electrode portion 32A, a potential corresponding to the pH of the supplied liquid in the vicinity of the electrode portion 32A. The potentiometer 32B measures the difference between the potential of the electrode portion 32A and the potential of the reference electrode 34 (FIG. 2) and outputs to the control section 120 a signal indicating the potential difference (a voltage signal corresponding to the pH of the supplied liquid).

The nitrite ion sensor 28 measures the concentration of nitrite ion or nitrous acid gas (measurement targets) contained in the supplied liquid after addition of an acid thereto from the containing section 176A. The nitrite ion sensor 28 is provided in the supply passage 130 to be located on the downstream side of the merging portion 130C and on the downstream side of the pH sensor 32 (specifically, on the downstream side of the ammonium ion sensor 26). Notably, when the control section 120 measures the concentration of nitrous acid gas by using the nitrite ion sensor 28, the control section 120 brings the electromagnetic valve 166 into its open state, thereby permitting flow of liquid between the supply passage 182 and the supply passage 130. Furthermore, the control section 120 brings the three-way valve 168 into its first changeover state, thereby permitting flow of liquid between the containing section 176A and the supply passage 180, and activates the pump 154. As a result of these operations, the acid contained in the containing section 176A is suppled to the supply passage 130, so that nitrite ion contained in the supplied liquid can be transformed into nitrous acid, which forms a gas. In the water quality measurement device 100, when an acid is added to the supplied liquid, adjustment is made such that the pH of the acid-added liquid (the supplied liquid on the downstream side of the merging portion 130C) becomes 1.5 or lower.

The nitrite ion sensor 28 (nitrous acid sensor) is configured in the form of a well-known diaphragm-type ion sensor and includes an ion electrode 28A, a third potentiometer 28B, and a reference electrode 28C. The ion electrode 28A is configured in the form of a well-known diaphragm-type ion electrode. The ion electrode 28A is configured, for example, such that nitrous acid gas passes through its diaphragm and dissolves into electrolytic solution inside the electrode, and an electromotive force corresponding to the pH of the internal solution which changes due to nitrous acid gas is generated. In the manner as described above, the ion electrode 28A can detect nitrous acid gas selectively. By virtue of this, it becomes possible to prevent ions (interfering ions) still contained in the water from adversely affecting the detection of nitrous acid, thereby accurately measuring the concentration of nitrous acid. The ion electrode 28A is immersed in the supplied liquid flowing through the supply passage 130 on the downstream side of the merging portion 130C. The ion electrode 28A generates an electrode potential corresponding to the concentration of nitrous acid gas contained in the supplied liquid having passed the merging portion 130C (namely, nitrous acid gas composed of nitrous acid gas that has been contained, from the beginning, in the supplied liquid before passing the merging portion 130C and nitrous acid gas formed from nitrite ion as a result of passage of the merging portion 130C). The third potentiometer 28B is electrically connected to the ion electrode 28A and the reference electrode 28C. The third potentiometer 28B measures the difference between the electrode potential of the ion electrode 28A and the electrode potential of the reference electrode 28C and outputs to the control section 120 a signal indicating that potential difference (a signal indicating a potential difference corresponding to the concentration of nitrous acid gas composed of nitrous acid gas contained in the breeding water from the beginning and nitrous acid gas formed from nitrite ion).

The ammonium ion sensor 26 (ammonia sensor) measures the concentration of ammonium ion or ammonia (measurement targets) contained in the supplied liquid after addition of a base thereto from the containing section 176B. The ammonium ion sensor 26 is provided in the supply passage 130 to be located on the downstream side of the merging portion 130C and on the downstream side of the pH sensor 32. Notably, when the control section 120 measures the concentration of ammonia gas by using the ammonium ion sensor (ammonia sensor) 26, the control section 120 brings the electromagnetic valve 166 into its open state, thereby permitting flow of liquid between the supply passage 182 and the supply passage 130. Furthermore, the control section 120 brings the three-way valve 168 into its second changeover state, thereby permitting flow of liquid between the containing section 176B and the supply passage 180, and activates the pump 154. As a result of these operations, the base contained in the containing section 176B is suppled to the supply passage 130, so that ammonium ion contained in the supplied liquid can be transformed into ammonia, which forms a gas. In the water quality measurement device 100, when a base is added to the supplied liquid, adjustment is made such that the pH of the base-added liquid (the supplied liquid on the downstream side of the merging portion 130C) becomes 11.5 or higher.

The ammonium ion sensor 26 is configured in the form of a well-known diaphragm-type ion sensor and includes an ion electrode 26A, a fourth potentiometer 26B, and a reference electrode 26C. The ion electrode 26A is configured in the form of a well-known diaphragm-type ion electrode. The ion electrode 26A is configured, for example, such that ammonia gas passes through its diaphragm and dissolves into electrolytic solution inside the electrode, and an electromotive force corresponding to the pH of the internal solution which changes due to ammonia gas is generated. In the manner as described above, the ion electrode 26A can detect ammonia gas selectively. By virtue of this, it becomes possible to prevent ions (interfering ions) still contained in the water from adversely affecting the detection of ammonia, thereby accurately measuring the concentration of ammonia. The ion electrode 26A is immersed in the supplied liquid flowing through the supply passage 130 on the downstream side of the merging portion 130C. The ion electrode 26A generates an electrode potential corresponding to the concentration of ammonia gas contained in the supplied liquid having passed the merging portion 130C (namely, ammonia gas composed of ammonia gas that has been contained, from the beginning, in the supplied liquid before passing the merging portion 130C and ammonia gas formed from ammonium ion as a result of passage of the merging portion 130C). The fourth potentiometer 26B is electrically connected to the ion electrode 26A and the reference electrode 26C. The fourth potentiometer 26B measures the difference between the electrode potential of the ion electrode 26A and the electrode potential of the reference electrode 26C and outputs to the control section 120 a signal indicating that potential difference (a signal indicating a potential difference corresponding to the concentration of ammonia gas composed of ammonia gas contained in the supplied liquid from the beginning and ammonia gas formed from ammonium ion). The supplied liquid having passed the ammonium ion sensor 26 is drained from the drain passage 134.

### 2. Regarding calibration liquid

The following description relates to the details of the calibration liquid 1.

The calibration liquid 1 is calibration liquid for a water quality measurement device. In the example of FIG. 1, the calibration liquid 1 is used for calibration of the water quality measurement device 100. Specifically, the calibration liquid 1 is used in a method for calibrating the concentration measuring characteristics of the water quality measurement device 100 for a plurality of types of target components. The calibration liquid 1 includes solutions which contain the plurality of types of target ions, whose concentrations are to be measured by the water quality measurement device 100, at respective concentrations determined for the respective types of target ions.

In the example of FIG. 1, the calibration liquid 1 includes a first solution 1A and a second solution 1B. The first solution 1A and the second solution 1B contain a plurality of types of common target ions. In the example of FIG. 1, the plurality of types of target ions, whose concentrations are to be measured by the water quality measurement device 100, are calcium ion, magnesium ion, nitrite ion, and ammonium ion. Accordingly, the first solution 1A contains calcium ion, magnesium ion, nitrite ion, and ammonium ion at respective concentrations determined for the respective types of target ions. Similarly, the second solution 1B contains calcium ion, magnesium ion, nitrite ion, and ammonium ion at respective concentrations determined for the respective types of target ions.

As to the target ions commonly contained in the first solution 1A and the second solution 1B, their concentrations differ between the first solution 1A and the second solution 1B. For example, the calcium ion concentration of the first solution 1A differs from the calcium ion concentration of the second solution 2A. The magnesium ion concentration of the first solution 1A differs from the magnesium ion concentration of the second solution 2A. Furthermore, the nitrite ion concentration of the first solution 1A differs from the nitrite ion concentration of the second solution 2A. The ammonium ion concentration of the first solution 1A differs from the ammonium ion concentration of the second solution 2A. Furthermore, the pH of the first solution 1A differs from the pH of the second solution 1B. The pH of the first solution 1A and the pH of the second solution 1B are set to fall within a predetermined pH range.

The plurality of types of target ions contained in the calibration liquid 1 include one or more types of gas formation ions. Gas formation ion is a type of ion whose transformation to a gas is promoted when the pH of liquid containing the ion falls outside a pH stable range determined for the ion. In the above-described example in which the solutions contain four types of ions, ammonium ion and nitrite ion are gas formation ions. The pH of the first solution 1A is set to fall within the pH stable ranges of all the gas formation ions (ammonium ion and nitrite ion) contained in the first solution 1A. The pH stable range of ammonium ion is a pH range lower than 6.5. The pH stable range of nitrite ion is a pH range higher than 5.5. Accordingly, when the value of the pH of the first solution 1Ais represented by X1ph, a relation of 5.5 < X1ph < 6.5 is satisfied. Similarly, the pH of the second solution 1B is set to fall within the pH stable ranges of all the gas formation ions (ammonium ion and nitrite ion) contained in the second solution 1B. Accordingly, when the value of the pH of the second solution 1B is represented by X2ph, a relation of 5.5 < X2ph < 6.5 is satisfied.

The plurality of types of target ions contained in the calibration liquid 1 include one or more types of compound formation ions. Compound formation ion is a type of ion whose transformation to a compound is promoted when the pH of liquid containing the ion falls outside a pH stable range determined for the ion. In the above-described example in which the solutions contain four types of ions, calcium ion and magnesium ion are compound formation ions. The pH of the first solution 1A is set to fall within the pH stable ranges of all the compound formation ions (calcium ion and magnesium ion) contained in the first solution 1A. The pH stable range of calcium ion is a pH range lower than 12.8. The pH stable range of magnesium ion is a pH range lower than 11.6. Accordingly, when the value of the pH of the first solution 1A is represented by X1ph, a relation of X1ph < 11.6 is satisfied. Similarly, the pH of the second solution 1B is set to fall within the pH stable ranges of all the compound formation ions (calcium ion and magnesium ion) contained in the second solution 1B. Accordingly, when the value of the pH of the second solution 1B is represented by X2ph, a relation of X2ph < 11.6 is satisfied.

Both the first solution 1A and the second solution 1B satisfy conditions regarding the above-described pH range desirable for the gas formation ions and the above-described pH range desirable for the compound formation ions. Accordingly, the value X1ph of the pH of the first solution 1A is between 5.5 and 6.5, and the value X2ph of the pH of the second solution 2A is between 5.5 and 6.5. The pH of the first solution 1A and the pH of the second solution 2A are set such that their pH values differ from each other and satisfy the above-described pH-range conditions.

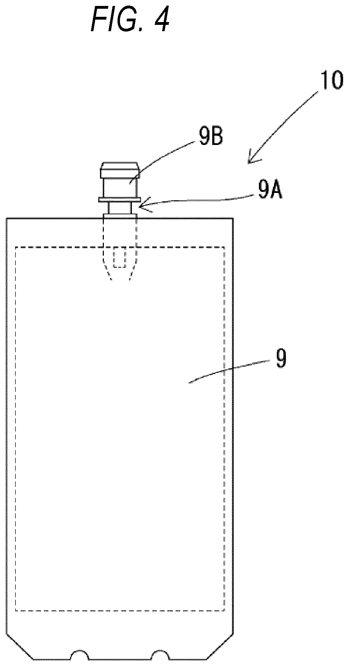

Each of the first solution 1A and the second solution 1B of the calibration liquid 1 can be contained in a container body 9 as shown in FIG. 4 and be managed. The first solution 1A is contained in a container body 9 in a tightly sealed state and is managed. When this container body 9 is used, this container body 9 is connected to the water quality measurement device 100 and serves as the containing section 172. The second solution 1B is contained in a container body 9 different from the above-mentioned container body 9 in a tightly sealed state and is managed. When this container body 9 is used, this container body 9 is connected to the water quality measurement device 100 and serves as the containing section 174. An example connection structure between the water quality measurement device 100 and the respective container bodies 9 is a connection method in which respective tubes provided on the upstream side of the electromagnetic valves 142 and 144 (a tube which constitutes a pipe line on the upstream side of the electromagnetic valve 142 and a tube which constitutes a pipe line on the upstream side of the electromagnetic valve 144) are individually inserted into the respective container bodies 9, and end portions of the respective tubes are disposed in the first solution 1A and the second solution 1B within the respective container bodies 9.

Each container body 9 is, for example, a pouch-type container and has a structure which enables the calibration liquid 1 to be contained in the pouch in a tightly sealed manner. Each container body 9 has, for example, a structure in which two pieces of sheet or film material formed of aluminum, plastic film, vapor deposition film, or the like are superposed on each other, and their peripheral edge portions are bonded together. Each container body 9 has an opening portion 9A through which a space inside the container body 9 and a space outside the container body 9 communicate with each other. A removable cap portion 9B is attached to the opening portion 9A. Each container body 9 has a structure which enables discharge of the calibration liquid 1 to the outside through the opening portion 9A. Also, each container body 9 has, for example, a structure which enables a tube or the like to be connected to the container body 9 via the opening portion 9A, thereby enabling the supply of the calibration liquid 1 to the water quality measurement device 100. An outer portion of the container body 9 may have light blocking properties and/or gas barrier properties.

The next description relates to effects of the calibration liquid 1.

The above-described calibration liquid 1 enables performance of calibration for the plurality of types of target components by using a common solution (the first solution 1A or the second solution 1B). Therefore, when the water quality measurement device 100 is calibrated, the calibration liquid 1 can reduce management burden resulting from preparation of dedicated calibration liquid for each target component. In addition, use of the calibration liquid 1 can easily shorten calibration time, as compared with a method in which dedicated calibration liquids for the respective target components are sequentially fed to the water quality measurement device 100 so as to sequentially perform calibration operations corresponding to the respective target components.

The calibration liquid 1 enables performance of calibration for the plurality of types of target components, and gas formation ions (ammonium ion and nitrite ion) can be included therein. In addition, in the calibration liquid 1, the pH of each solution is set to fall within the pH stable ranges of all types of gas formation ions contained in the solution. Therefore, the calibration liquid 1 is stably held in a state in which transformation of the gas formation ions to gases is suppressed.

The calibration liquid 1 enables performance of calibration for the plurality of types of target components, and compound formation ions (calcium ion and magnesium ion) can be included therein. In addition, in the calibration liquid 1, the pH of the solution is set to fall within the pH stable ranges of all types of compound formation ions contained in the solution. Therefore, the calibration liquid 1 is stably held in a state in which transformation of the compound formation ions to compounds is suppressed.

Specifically, the calibration liquid 1 contains the above-described compound formation ions and the above-described gas formation ions, and the pH of the solution is set to fall within the pH stable ranges of all types of compound formation ions and fall within the pH stable ranges of all types of gas formation ions. This calibration liquid 1 is stably held in a state in which transformation of the target components to gases and transformation of the target components to compounds are suppressed in an environment in which the gas formation ions and the compound formation ions coexist in the solution.

In the case where the water quality measurement device 100 performs measurement for the first solution 1A of the calibration liquid 1, the water quality measurement device 100 can accurately specify the relation between the concentration of each of the target components contained in the first solution 1A and an electrical signal (for example, a voltage signal) output from the corresponding sensor. In the case where the water quality measurement device 100 performs measurement for the second solution 1B, the water quality measurement device 100 can accurately specify the relation between the concentration of each of the target ions contained in the second solution 1B and an electrical signal (for example, a voltage signal) output from the corresponding sensor. Therefore, the calibration liquid 1 enables the water quality measurement device 100 to accurately obtain a relational expression representing the relation between the concentration of each target component and the corresponding electrical signal.

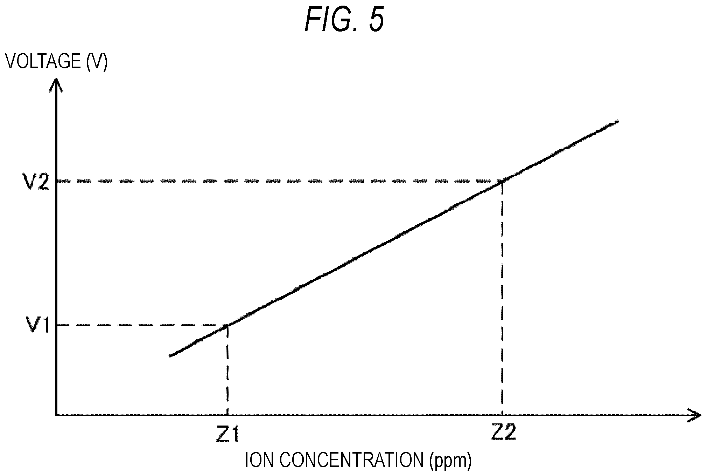

For example, in the case where, as shown in FIG. 5, a detection value V1 is obtained when the calcium ion (concentration Z1) contained in the first solution 1A is measured by the calcium ion sensor 22 and a detection value V2 is obtained when the calcium ion (concentration Z2) contained in the second solution 2A is measured by the calcium ion sensor 22, from these values, a linear relational expression as shown in FIG. 5 can be obtained as the relational expression of the calcium ion sensor 22. For other target ions, relational expressions can be obtained in the same manner.

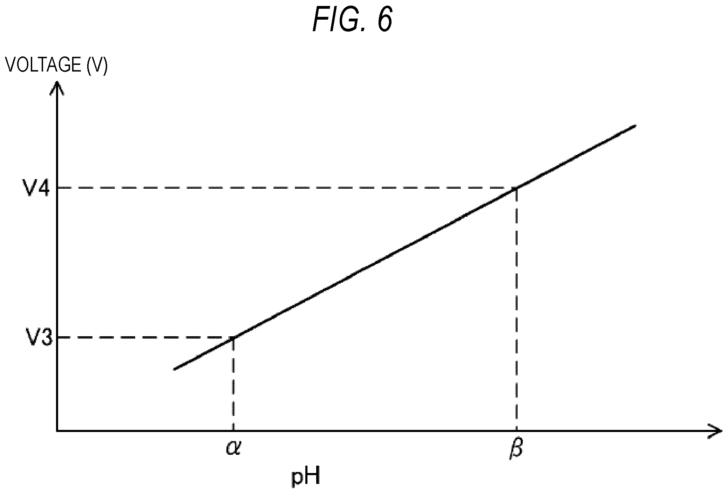

Also, in the case where, as shown in FIG. 6, a detection value V3 is obtained when the first solution 1A (pH: α) is measured by the pH sensor 30 and a detection value V4 is obtained when the second solution 1B (pH: β) is measured by the pH sensor 30, from these values, a linear relational expression as shown in FIG. 6 can be obtained as the relational expression of the pH sensor 30.

The solutions (the first solution 1A and the second solution 1B) of the calibration liquid 1 may contain at least sodium ion, chloride ion, and potassium ion. In this case, the target ions contain ions other than sodium ion, chloride ion, and potassium ion (calcium ion, magnesium ion, nitrite ion, and ammonium ion). This calibration liquid 1 enables calibration of a water quality measurement device 100 which measures the concentrations of a plurality of types of target ions contained in a solution which contains seawater or components of seawater; specifically, calibration of the water quality measurement device 100 for the plurality of types of target ions.

The calibration-liquid-filled container 10 includes the calibration liquid 1 and the container body 9 for containing the calibration liquid 1, and the calibration liquid 1 is contained in the container body 9 in a tightly sealed manner. By virtue of this calibration-liquid-filled container 10, the calibration liquid 1 containing a plurality of types of ions in a mixed state can be stably managed in the container body 9, which is high in sealing performance.

### <Other embodiments>

The present invention is not limited to the embodiment described by the above description with reference to the drawings. For example, the features of the above-described embodiment and embodiments which will be described below can be combined in any manner so long as no contradiction occurs. Also, any of the features of the above-described embodiment and embodiments which will be described below may be omitted unless explicitly stated as essential. Moreover, the above-described embodiment may be modified as follows.

In the above-described embodiment, ammonium ion and nitrite ion are mentioned as examples of gas formation ions. However, carbonate ion may be contained as a gas formation ion. The pH stable range of carbonate ion is a pH range higher than 9. Accordingly, when carbonate ion is added to the first solution and the second solution, the pH values of these solutions are desirably set to fall within the pH range higher than 9.

In the above-described embodiment, calcium ion and magnesium ion are mentioned as examples of compound formation ions. However, phosphate ion, boron, sodium ion, potassium ion, ferric iron, manganese ion, zinc ion, copper ion, molybdenum ion, iodine ion, chloride ion, sulfide ion, nitrate ion, etc. may be contained as compound formation ions. In the case where phosphate ion is contained, desirably, the pH of each solution of the calibration liquid falls within the stable range of phosphate ion (a range higher than pH 2.2). In the case where boron is contained, desirably, the pH of each solution of the calibration liquid falls within the stable range of boron ion (a range higher than pH 9.2). In the case where sodium ion is contained, desirably, the pH of each solution of the calibration liquid falls within the stable range of sodium ion (a range lower than pH 13). In the case where potassium ion is contained, desirably, the pH of each solution of the calibration liquid falls within the stable range of potassium ion (a range lower than pH 13). In the case where ferric ion is contained, desirably, the pH of each solution of the calibration liquid falls within the stable range of ferric ion (a range lower than pH 5). In the case where manganese ion is contained, desirably, the pH of each solution of the calibration liquid falls within the stable range of manganese ion (a range lower than pH 10.6). In the case where zinc ion is contained, desirably, the pH of each solution of the calibration liquid falls within the stable range of zinc ion (a range lower than pH 7.8). In the case where copper ion is contained, desirably, the pH of each solution of the calibration liquid falls within the stable range of copper ion (a range lower than pH 7.4). In the case where molybdenum ion is contained, desirably, the pH of each solution of the calibration liquid falls within the stable range of molybdenum ion (a range lower than pH 12.8). In the case where iodine ion is contained, desirably, the pH of each solution of the calibration liquid falls within the stable range of iodide ion (a range lower than pH 10.6). In the case where chloride ion is contained, desirably, the pH of each solution of the calibration liquid falls within the stable range of chloride ion (a range lower than pH 12.5). In the case where sulfide ion is contained, desirably, the pH of each solution of the calibration liquid falls within the stable range of chloride ion (a range lower than pH 12.5).

In the above-described embodiment, the water quality measurement device 100 is configured to introduce an acid and a base from the common passage. However, the water quality measurement device 100 may be configured such that an acid and a base are selectively introduced via different passages.

In the above-described embodiment, the calibration liquid 1 includes the first solution and the second solution. However, the calibration liquid may further include a third solution which contains a plurality of types of target ions identical to those of the first solution and the second solution. The calibration liquid may include four or more types of solutions which contain the same target ions. Each of the plurality of types of target ions commonly contained in the plurality of types of solutions may differ in ion concentration among the solutions. The pH values of the plurality of types of solutions may differ from one another.

"Water used for product growth management" is mentioned as an example of the measurement target liquid, which is the measurement target of the water quality measurement device 100. However, its specific example is not limited to the above-described example. For example, a specific example of the "product growth management" may be "growth management in fisheries," "growth management in agriculture," "growth management in the livestock industry," "growth management in forestry," or the like. Alternatively, the "product growth management" may be growth management in other primary industries.

Specifically, the measurement target liquid, which is the measurement target of the water quality measurement device 100, may be water used for "management of aquaculture of an aquatic organism." In this case, an example of the "water used for management of aquaculture of an aquatic organism" is breeding water used for breeding the aquatic organism. Also, an example of the "aquatic organism" is a shellfish such as prawn. However, the aquatic organism is not limited to this example and may be an organism (other than prawn) of *Decapoda*, *Malacostraca*, *Crustacea*, or *Arthropoda.* Alternatively, the aquatic organism may be fishes such as sea bream, shellfishes such as scallop, or seaweeds such as *Wakame* seaweed.

Notably, the embodiments disclosed this time should be considered to be illustrative and not to be restrictive in all aspects. The scope of the present invention is not limited to the embodiments disclosed this time, and it is intended that the present invention encompasses all modifications within the range shown by the claims and the range of equivalents of the claims.

### REFERENCE SIGNS LIST

1: calibration liquid
1A: first solution
1B: second solution
9: container body
10: calibration-liquid-filled container
100: water quality measurement device

## Claims

1. A calibration liquid for water quality measurement device which is used for calibration of a water quality measurement device which includes a plurality of sensors to be brought into contact with a measurement target liquid so as to measure concentrations of a plurality of types of target components contained in the measurement target liquid and which measures the concentrations of the plurality of types of target components, the calibration liquid being used for calibrating characteristics of the water quality measurement device for the concentrations of the plurality of types of target components,
wherein the calibration liquid includes a solution which contains the plurality of types of target components at respective concentrations determined for the target components, and
the solution has a pH within a predetermined pH range.

2. The calibration liquid for water quality measurement device according to claim 1,
wherein one or more types of target components among the plurality of types of target components are gas formation ions for which pH stable ranges are determined such that, when the pH of liquid containing each gas formation ion falls outside the corresponding pH stable range, transformation of the gas formation ion to a gas is promoted, and
the pH of the solution is set to fall within the pH stable ranges of all types of the gas formation ions contained in the solution.

3. The calibration liquid for water quality measurement device according to claim 1 or 2,
wherein one or more types of target components among the plurality of types of target components are compound formation ions for which pH stable ranges are determined such that, when the pH of liquid containing each compound formation ion falls outside the corresponding pH stable range, transformation of the compound formation ion to a compound is promoted, and
the pH of the solution is set to fall within the pH stable ranges of all types of the compound formation ions contained in the solution.

4. The calibration liquid for water quality measurement device according to any one of claims 1 to 3,
wherein the solution includes at least a first solution and a second solution which are different solutions,
the first solution and the second solution contain the plurality of types of target components as common target components,
concentrations of the common target components contained in the first solution differ from concentrations of the common target components contained in the second solution, and
the pH of the first solution and the pH of the second solution differ from each other.

5. The calibration liquid for water quality measurement device according to any one of claims 1 to 4,
wherein the solution contains at least sodium ion, chloride ion, and potassium ion, and the target components include components other than sodium ion, chloride ion, and potassium ion.

6. A calibration-liquid-filled container for water quality measurement device, comprising:
the calibration liquid for water quality measurement device according to any one of claims 1 to 5; and
a container body for containing the calibration liquid,
wherein the calibration liquid is contained in the container body, and the container body is tightly sealed.

7. A calibration method for a water quality measurement device which measures concentrations of a plurality of types of target components contained in a measurement target liquid, the calibration method calibrating characteristics of the water quality measurement device for the concentrations of the plurality of types of target components,
wherein the calibration liquid for water quality measurement device according to any one of claims 1 to 5 is used.
